# EUROPÄISCHE PATENTSCHRIFT

(11) **EP 0 533 132 B1**
(45) Veröffentlichungstag und Bekanntmachung des Hinweises auf die Patenterteilung: **02.11.1994**
(21) Anmeldenummer: 92115833.3
(22) Anmeldetag: 16.09.1992
(51) Int. Cl.: C07D 213/79

(54) **Verfahren zur Oxidation von Hydroxymethylpyridinderivaten zu Pyridincarbonsäurederivaten**
Process for the oxidation of hydroxymethylpyridine derivatives to pyridine carboxylic acid derivatives
Procédé d'oxydation de dérivés de hydroxyméthyl pyridine en dérivés d'acide pyridine carboxylique

(30) Priorität: 19.09.1991 DE 4131220
(43) Veröffentlichungstag der Anmeldung: 24.03.1993
(73) Patentinhaber: HOECHST AKTIENGESELLSCHAFT, 65926 Frankfurt am Main (DE)
(72) Erfinder: Scharbert, Bernd, Dr., W-6230 Frankfurt am Main 71 (DE)

(56) Entgegenhaltungen:
- US-A- 3 291 805
- US-A- 4 482 439
- CHEMICAL ABSTRACTS, vol. 98, no. 20, 16. Mai 1983, Columbus, Ohio, US; abstract no. 169227p, D.C. TRIVEDI ET AL. 'Transition metal oxide anodes and their application in the synthesis of organic compounds.' Seite 535 ;

## Beschreibung

Die Erfindung betrifft ein Verfahren zur Oxidation von ortho-Hydroxymethylpyridinderivaten zu Pyridincarbonsäurederivaten.

Es ist bekannt, daß sich Hydroxymethylpyridine mit KMnO₄ zu den entsprechenden Carbonsäuren oxidieren lassen (DD-A-248 119). In vielen weiteren Verfahren ist die Reaktion von Hydroxymethylpyridinen mit Permanganat die Oxidationsmethode der Wahl [Pharmazie 39 (1984) 155, BE-A-866 977, JP-A-063 434, US-A-3,291,805]. Für eine industrielle Anwendung hat diese Methode allerdings gravierende Nachteile. So ist KMnO₄ ein teures Oxidationsmittel und nach der Reaktion ist der angefallene Braunstein zu entsorgen.

In einem anderen Verfahren wird Salpetersäure in konzentrierter Schwefelsäure als Oxidationsmittel eingesetzt. Nachteil in diesen Verfahren ist, daß die Reaktionsbedingungen drastisch sein müssen (15 h bei 160 - 180°C) und die gesamte Reaktionslösung zur Aufarbeitung auf pH 2 gebracht werden muß, wobei eine sehr hohe Salzfracht anfällt.

Weiterhin gibt es Verfahren, in denen in einer Zweistufenoxidation über den entsprechenden Aldehyd die Carbonsäure hergestellt wird. Hierbei werden allerdings teure und toxische Reagenzien wie Selendioxid oder Bleitetraacetat verwendet. Eine Übertragung dieser Methoden auf industrielle Maßstäbe ist deshalb nicht gegeben.

Die US-A-4,482,439 lehrt in Beispiel 14 die elektrochemische Oxidation von 4-Hydroxymethylpyridin an PbO₂-Anoden in einer durch eine Ionenaustauschermembran geteilten Zelle. Mit diesem Verahren ist es aber nicht möglich, eine Oxidation selektiv an der Hydroxymethylfunktion durchzuführen, ohne dabei andere Substituenten am Pyridinring mit zu oxidieren.

Es war deshalb die Aufgabe der vorliegenden Erfindung, ein Verfahren zur selektiven Oxidation von ortho-Hydroxymethylpyridinderivaten zu den entsprechenden Pyridincarbonsäurederivaten bereitzustellen, welches technisch einfach durchzuführen ist und das gewünschte Produkt in guten Ausbeuten hervorbringt. Die Selektivität des Verfahrens soll sich darin zeigen, daß andere Substituenten am Pyridinring wie Alkyl oder Alkoxogruppen nicht gleichzeitig oxidiert werden.

Die Aufgabe wird durch ein Verfahren gelöst, in dem ein Hydroxymethylpyridinderivat der allgemeinen Formel I 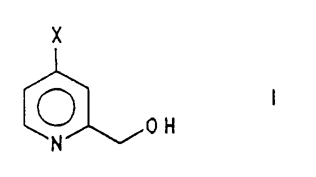
wobei
- X: Wasserstoff, (C₁-C₁₂)-Alkyl, (C₆-C₁₂)-Aryl, COOR¹, CONH₂, CONHR¹, CON(R¹)₂, CN, C(O)R¹, SO₂R¹, SO₃H, NO₂, worin
- R¹: Wasserstoff, (C₁-C₁₂)-Alkyl, (C₆-C₁₂)-Aryl,
bedeutet,
in eine Verbindung der Formel II 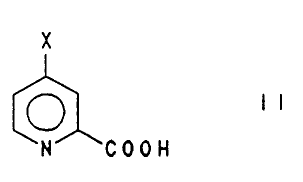
umgesetzt wird, das dadurch gekennzeichnet ist, daß die Verbindung gemäß Formel I elektrochemisch an NiO(OH)-Anoden oxidiert wird.

Bevorzugt ist das erfindungsgemäße Verfahren zur Oxidation der Verbindung der allgemeinen Formel I, in denen X Wasserstoff, (C₁-C₁₂)-Alkyl, (C₆-C₁₂)-Aryl, COOR¹, CONH₂, CONHR¹, CON(R¹)₂ und C(O)R¹ bedeuten.

Insbesondere bevorzugt werden Verbindungen der allgemeinen Formel I oxidiert, in denen X Wasserstoff, (C₁-C₆)-Alkyl, Phenyl oder Naphthyl, COOR¹, CONH₂, CONHR¹, CON(R¹)₂ und C(O)R¹ bedeuten.

R¹ steht bevorzugt für Wasserstoff, (C₁-C₆)-Alkyl, Phenyl oder Naphthyl.

Das Material der Anodenelektrode besteht aus Kohle oder Metall, und ist jeweils mit einer Schicht aus NiO(OH) bedeckt ist. Von den Metallelektroden sind bevorzugt Stahl- oder Nickelelektroden, die mit NiO(OH) überzogen sind.

Die Formierung der NiO(OH)-Schicht erfolgt dabei so, daß aus einer wäßrigen Lösung löslicher Nickelsalze mit einer Konzentration an Nickel-Ionen zwischen 0,01 und 2 Mol/l, bevorzugt zwischen 0,05 und 1 Mol/l, auf die genannten Elektrodenmaterialien Nickel kathodisch abgeschieden, anschließend die Nickelsalzlösung gegen eine wäßrige alkalische Lösung mit einer Konzentration an Alkalihydroxid zwischen 0,001 bis 2 Mol/l, bevorzugt zwischen 0,01 und 1 Mol/l, ausgetauscht wird und die genannten Elektrodenmaterialien mit dem abgeschiedenen Nickel anodisch polarisiert werden.

Sowohl die kathodische Nickelabscheidung als auch die anodische Polarisierung vor der elektrodischen Oxidation der Verbindung gemäß Formel I erfolgen 5 bis 120 Minuten lang, bevorzugt 20 bis 40 Minuten lang bei einer Stromdichte zwischen 1 und 100 mA/cm², bevorzugt zwischen 2 und 10 mA/cm².

Das Material der Kathodenelektrode soll gewährleisten, daß sich unter den vorgegebenen Elektrolysebedingungen Wasserstoff entwickelt. Bevorzugt hierfür sind Nickel, Palladium, Platin oder Stahl.

Grundsätzlich kann das erfindungsgemäße Verfahren in einer geteilten oder ungeteilten Elektrodenzelle durchgeführt werden. Bevorzugt ist das Verfahren in einer ungeteilten Zelle. Sofern eine geteilte Zelle verwendet wird, kommen zur Teilung der Zelle alle Arten von Ionenaustauschermembranen oder auch anorganische Werkstoffe in Frage.

Als Elektrolyt (in der ungeteilten Zelle) bzw. Anolyt (in der geteilten Zelle) werden wäßrige Lösungen verwendet, die 0,001 bis 2 Mol/l, bevorzugt zwischen 0,01 bis 1 Mol/l Alkalihydroxid, bevorzugt NaOH enthalten. Die Konzentration an Verbindung gemäß Formel I liegt zwischen 0,001 und 10 Mol/l, bevorzugt zwischen 0,01 und 0,5 Mol/l.

Die Elektrolysetemperatur liegt zwischen 5 und 100°C, bevorzugt zwischen 15 und 70°C, die Stromdichte zwischen 1 und 50 mA/cm², bevorzugt zwischen 2 und 20 mA/cm².

Die Elektrolyse kann bis zur vollständigen Umsetzung gefahren werden. Der Umsatz kann über HPLC-Analytik kontrolliert werden. Es ist möglich, aber nicht bevorzugt, die Elektrolyse bei unvollständigem Umsatz abzubrechen und anschließend Edukt und Produkt zu trennen.

Die Aufarbeitung erfolgt dadurch, daß bis zu dem isoelektrischen Punkt der entsprechenden Carbonsäure neutralisiert wird. Die Carbonsäure fällt dabei aus. Noch etwas höhere Ausbeuten erhält man, wenn das Elektrolysat über ein stark saures Ionenaustauscherharz filtriert wird. Das wäßrige Filtrat enthält reine Verbindung gemäß Formel II. Um weitere auf dem Harz adsorbierte Verbindungen gemäß Formel II zu gewinnen, wird das Harz anschließend mit Methanol gewaschen. Das Methanol-Filtrat und das wäßrige Filtrat werden jeweils zur Trockne eingedampft und ergeben vereinigt eine hohe Ausbeute an Verbindung gemäß Formel II.

Enthält die Verbindung gemäß Formel I eine Esterfunktion (X = COOR¹), so ist diese unter den Elektrolysebedingungen nicht stabil und muß vor der Elektrolyse verseift, sowie der freiwerdende Alkohol R¹OH entfernt werden. Eine Verseifung während der Elektrolyse hätte zur Folge, daß der freiwerdende Alkohol R¹OH ebenso wie die Verbindung gemäß Formel I an der NiO(OH)-Anode oxidiert würde. Die Oxidation des Alkohols R¹OH würde gegen die Oxidation der Verbindung gemäß Formel I konkurrieren. Eine Erniedrigung der Stromausbeute wäre die Folge.

Bei der Verseifung wird so verfahren, daß die für die Elektrolyse angesetzte wäßrige alkalische Lösung für 1 bis 60 Minuten, bevorzugt 5 bis 10 Minuten, auf eine Temperatur zwischen 50 und 100°C, bevorzugt zwischen 70 und 90°C gebracht wird.

Die wäßrige alkalische Lösung wird mit einem organischen Lösemittel, bevorzugt Dichlormethan oder Essigsäureethylester, extrahiert, und die wäßrige Phase bereitgehalten. Für Alkohole R¹OH mit einem Siedepunkt kleiner als 100°C können auch zwischen 1 und 10 Vol.-%, bevorzugt zwischen 3 und 5 Vol.-% der Lösung abgedampft werden. In diesem Fall ist im entfernten Anteil der Alkohol-Anteil enthalten. Für Alkohole R¹OH mit einem Siedepunkt kleiner als 100°C ist die letztgenannte Methode bevorzugt.

Die zurückbleibende Losung enthält bei dieser Vorgehensweise, unabhängig von den zuvor genannten Varianten, die Verbindung gemäß Formel I mit X = COOH, bzw. im alkalischen Milieu COO⁻M⁺ (M = Alkalimetall), keinen freien Alkohol R¹OH mehr. Sie kann für die Elektrolyse eingesetzt werden.

Das erfindungsgemäße Verfahren zeichnet sich dadurch aus, daß Verbindungen gemäß Formel I nahezu quantitativ umgesetzt werden. Die Herstellung der Ausgangsmaterialien gemäß der allgemeinen Formel I wird in der gleichzeitig eingereichten deutschen Patentanmeldung DE-A-41 31 217 beschrieben. Die Reaktionsbedingungen und die Aufarbeitung sind technisch leicht durchführbar.

Bei der erfindungsgemäßen Oxidation werden die Substituenten X am Pyridinring nicht angegriffen. Insbesondere bei Verbindungen mit den oxidierbaren Substituenten X = C(O)R¹ und R¹ läßt sich das elektrochemische Verfahren des Standes der Technik an PbO₂-Anoden nicht anwenden, da diese Substituenten oxidativ umgewandelt würden. Desweiteren wurde festgestellt, daß die Verbindung gemäß Formel I mit X = COOR¹ nach dem beschriebenen Verfahren des Standes der Technik nur in Ausbeuten kleiner 70 % gebildet wird (Vergleichsbeispiel A). Nach dem erfindungsgemäßen Verfahren werden überraschenderweise Ausbeuten von größer als 96 % (HPLC-Analytik) und isolierte Ausbeuten größer als 90 % erreicht. Weiterhin ist hervorzuheben, daß nach dem Verfahren des Standes der Technik die Verbindung gemäß Formel I mit X = COOR¹ während der Elektrolyse langsam verseift und somit ein Gemisch der Produkte gemaß Formel II mit X = COOH bzw. COOR¹ anfällt.

Beispielsweise ist Pyridin-2,4-dicarbonsäure nach dem erfindungsgemäßen Verfahren aus der Verbindung gemaß Formel I mit X = COOR¹ darstellbar. Verbindungen dieses Typs sind gemäß DE-A-3,432,094 Vorprodukte für Verbindungen, die zur Inhibierung der Prolin- und Lysinhydroxylase verwendet werden.

Die Erfindung soll anhand der nachfolgend aufgeführten Beispiele näher erläutert werden.

Formierung der Anode:
In einer Zelle mit zwei sich gegenüberliegenden, planaren und gleichgroßen Elektroden, eine Elektrode aus Nickel, die andere aus Edelstahl, bei denen jeweils die Rückseite mit Glas abgedeckt ist, wird unter kathodischer Polarisierung der Nickelelektrode 30 Minuten lang in ungeteilter Zelle bei einer Stromdichte von 10 mA/cm² in einer 0,2 M Ni(NO₃)₂-Lösung elektrolysiert. Danach wird der Elektrolyt gegen eine 0,1 N NaOH-Lösung ausgetauscht und unter anodischer Polarisierung der Nickelelektrode wie oben beschrieben 30 Minuten lang elektrolysiert. Ein einheitlich schwarzer Belag zeigt die Bildung von NiO(OH) an. Bis zum Einsatz der Elektrode wird ein kleiner Schutzstrom im Bereich von 1 mA/cm² aufrecht erhalten. Vor Verwendung für eine elektrochemische Oxidation wird die Elektrode kurz mit Wasser gespült.

### Beispiel 1

1,67 g (0,01 Mol) 2-Hydroxymethyl-4-methoxycarbonyl-pyridin werden in 300 ml 1 N Natronlauge gelöst. Bei 90°C werden 10 ml der Lösung abgedampft und die zurtickbleibende Lösung in eine ungeteilte Glastopfzelle mit einer NiO(OH)-Anode und einer Edelstahl-Kathode eingefüllt. Bei einer Temperatur von 25°C und einer Stromdichte von 10 mA/cm² wird elektrolysiert. Mittels HPLC wird die Ausbeute an Pyridin-2,4-dicarbonsäure bestimmt.
Bei Durchgang von 4 F/Mol beträgt die Ausbeute 50 %.
Bei Durchgang von 6 F/Mol beträgt die Ausbeute 63 %.
Bei Durchgang von 8 F/Mol beträgt die Ausbeute 73 %.

### Beispiel 2

Dieses Beispiel entspricht Beispiel 1, nur beträgt die Stomdichte während der Elektrolyse 2,5 mA/cm².
Bei Durchgang von 2,7 F/Mol beträgt die Ausbeute 38 %.
Bei Durchgang von 8,5 F/Mol beträgt die Ausbeute 84 %.
Bei Durchgang von 11,8 F/Mol beträgt die Ausbeute 96 %.

### Beispiel 3

Dieses Beispiel entspricht Beispiel 1, mit der Ausnahme, daß die Elektrolyse bis zu einem Durchsatz von 8 F/Mol bei einer Stromdichte von 10 mA/cm² betrieben, danach bei einer Stromdichte von 2,5 mA/cm² bis zu einem Durchsatz von 12 F/Mol weiterelektrolysiert wird. Das Elektrolysat wird über einen stark sauren Ionenaustauscher filtriert. Das Eluat ergibt nach dem Gefriertrocknen 0,83 g Pyridin-2,4-dicarbonsäure. Das Ionentauscherharz wird mit Methanol gewaschen und das Eluat abgedampft. Im Rückstand verbleiben 0,70 g Pyridin-2,4-dicarbonsäure. Die gesamte Ausbeute beträgt 9,2 mMol, entsprechend 92 %.

### Beispiel 4

Dieses Beispiel entspricht Beispiel 3, lediglich wird in anderer Weise aufgearbeitet. Das Elektrolysat nach der Elektrolyse wird mit 5 N HCl auf eine pH-Wert von 1,5 gebracht. Der ausgefallene Niederschlag wird abfiltriert und getrocknet. Es verbleiben 1,48 g (8,9 mMol) Pyridin-2,4-dicarbonsäure, entsprechend 89 %.

### Vergleichsbeispiel A

In einer durch eine Kationenaustauscher-Membran (®Nafion 324) geteilten elektrochemischen Zelle mit einer PbO₂-Schüttbett-Anode (Durchmesser 8 cm, Höhe 1 cm) und einer Platin-Katnode werden 1,67 g (0,01 Mol) 2-Hydroxymethyl-4-methoxycarbonyl-pyridin in 100 ml einer 14 %igen Schwefelsäure-Lösung bei einer Stromstarke von 1,0 A bei einer Temperatur von 25°C bis zu einem Stromverbrauch von 2,14 Ah (8 F/Mol) elektrolysiert. Der Katholyt besteht aus 7 %iger Schwefelsaure. Pyridin-2,4-dicarbonsäure wird mit Hilfe einer HPLC-Analytik in 63 % Ausbeute nachgewiesen, in 6 % Ausbeute liegt 2-Carboxy-4-methoxycarbonyl-pyridin vor. Die Ausgangsverbindung ist nur noch in Spuren von kleiner als 2 % vorhanden.

## Patentansprüche

1. Verfahren zur selektiven Oxidation von Hydroxymethylpyridinderivaten der allgemeinen Formel I 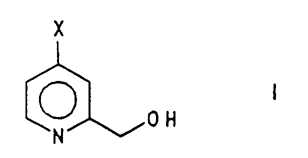 wobei
X Wasserstoff, (C₁-C₁₂)-Alkyl, (C₆-C₁₂)-Aryl, COOR¹, CONH₂, CONHR¹, CON(R¹)₂, CN, C(O)R¹, SO₂R¹, SO₃H, NO₂, worin
R¹ Wasserstoff, (C₁-C₁₂)-Alkyl, (C₆-C₁₂)-Aryl,
bedeutet,
in eine Verbindung der Formel II 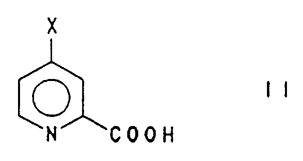 umgesetzt wird, dadurch gekennzeichnet, daß die Verbindung gemäß Formel I elektrochemisch an NiO(OH)-Anoden oxidiert wird.

2. Verfahren nach Anspruch 1, dadurch gekennzeichnet, daß Verbindungen der allgemeinen Formel I, in denen
X Wasserstoff, (C₁-C₁₂)-Alkyl, (C₆-C₁₂)-Aryl, COOR¹, CONH₂, CONHR¹, CON(R¹)₂ und C(O)R¹
bedeuten, oxidiert werden.

3. Verfahren nach den Ansprüchen 1 oder 2, dadurch gekennzeichnet, daß Verbindungen der allgemeinen Formel I, in denen
X Wasserstoff, (C₁-C₆)-Alkyl, Phenyl oder Naphthyl, COOR¹, CONH₂, CONHR', CON(R¹)₂ und C(O)R¹
bedeuten, oxidiert werden.

4. Verfahren nach den Ansprüchen 1 bis 3, dadurch gekennzeichnet, daß die Oxidation an einer Anode erfolgt, die aus Kohle oder einem Metall besteht, jeweils mit einer Schicht aus NiO(OH) belegt.

5. Verfahren nach den Ansprüchen 1 bis 4, dadurch gekennzeichnet, daß zur Ausbildung der NiO(OH)-Schicht vor der Elektrolyse aus einer wäßrigen Lösung löslicher Nickelsalze Nickel kathodisch auf dem Elektrodenmaterial abgeschieden werden.

6. Verfahren nach Anspruch 5, dadurch gekennzeichnet, daß anschließend die Nickelsalzlösung gegen eine wäßrige alkalische Lösung ausgetauscht wird und das Elektrodenmaterial anodisch polarisiert wird.

7. Verfahren nach Anspruch 6, dadurch gekennzeichnet, daß die Kathode aus Nickel, Palladium, Platin oder Stahl besteht.

8. Verfahren nach den Ansprüchen 1 bis 7, dadurch gekennzeichnet, daß die Oxidation in einer geteilten oder ungeteilten Elektrolysezelle durchgeführt wird.

9. Verfahren nach den Ansprüche 1 bis 8, dadurch gekennzeichnet, daß die kathodische Nickelabscheidung als auch die anodische Polarisierung 5 bis 120 Min, vorzugsweise 20 bis 40 Min bei einer Stromdichte zwischen 1 und 100 mA/cm², bevorzugt zwischen 2 und 10 mA/cm² durchgeführt wird.

10. Verfahren nach den Ansprüchen 1 bis 9, dadurch gekennzeichnet, daß als Elektrolyt bzw. Anolyt wäßrige Lösungen, die 0,001 bis 2 Mol/l Alkalihydroxid, enthalten, verwendet werden.

11. Verfahren nach den Ansprüchen 1 bis 10, dadurch gekennzeichnet, daß die Oxidation bei einer Elektrolysetemperatur zwischen 5 und 100°C und bei einer Stromdichte zwischen 1 und 50 mA/cm² durchgeführt wird.

## Claims

1. A process for selectively oxidising hydroxymethylpyridine derivatives of the formula I 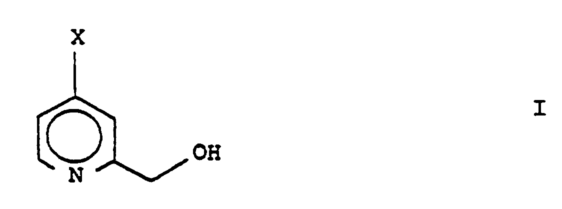 where
X is hydrogen, (C₁-C₁₂)alkyl, (C₆-C₁₂)aryl, COOR¹, CONH₂, CONHR¹, CON(R¹)₂, CN, C(O)R¹, SO₂R¹, SO₃H or NO₂, where
R¹ is hydrogen, (C₁-C₁₂) alkyl or (C₆-C₁₂) aryl,
is converted into a compound of the formula II 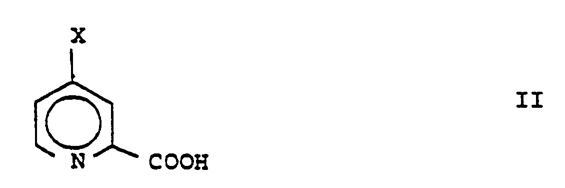 which process comprises oxidizing the compound represented by formula I electrochemically at NiO(OH) anodes.

2. The process as claimed in claim 1, wherein a compound of the formula I in which
X is hydrogen, (C₁-C₁₂)alkyl, (C₆-C₁₂)aryl, COOR¹, CONH₂, CONHR¹, CON(R¹)₂ or C(O)R¹
is oxidized.

3. The process as claimed in claim 1 or 2, wherein a compound of the formula I in which
X is hydrogen, (C₁-C₆)alkyl, phenyl or naphthyl, COOR¹, CONH₂, CONHR¹, CON(R¹)₂ or C(O)R¹
is oxidized.

4. The process as claimed in any of claims 1 to 3, wherein the oxidation is carried out at an anode which is composed of carbon or a metal, coated in all cases with a layer of NiO(OH).

5. The process as claimed in any of claims 1 to 4, wherein, for the formation of the NiO(OH) layer prior to the electrolysis, nickel is cathodically deposited on the electrode material from an aqueous solution of soluble nickel salts.

6. The process as claimed in claim 5, wherein the nickel salt solution is subsequently exchanged for an aqueous alkaline solution and the electrode material is anodically polarized.

7. The process as claimed in claim 6, wherein the cathode is composed of nickel, palladium, platinum or steel.

8. The process as claimed in any of claims 1 to 7, wherein the oxidation is carried out in a partitioned or unpartitioned electrolysis cell.

9. The process as claimed in any of claims 1 to 8, wherein the cathodic nickel deposition and the anodic polarization are carried out for 5 to 120 min, preferably 20 to 40 min, at a current density of between 1 and 100 mA/cm², preferably between 2 and 10 mA/cm².

10. The process as claimed in any of claims 1 to 9, wherein, as electrolyte or anolyte, aqueous solutions containing 0.001 to 2 mol/l of alkali-metal hydroxide are used.

11. The process as claimed in any of claims 1 to 10, wherein the oxidation is carried out at an electrolysis temperature of between 5 and 100°C and at a current density of between 1 and 50 mA/cm².

## Revendications

1. Procédé pour l'oxydation sélective de dérivés d'hydroxyméthylpyridine, dans lequel on convertit un dérivé d'hydroxyméthylpyridine de formule générale I 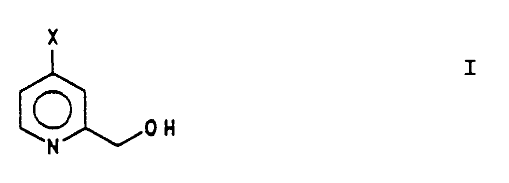 dans laquelle
X représente un atome d'hydrogène ou un groupe alkyle en C₁-C₁₂, aryle en C₆-C₁₂, COOR¹, CONH₂, CONHR¹, CON(R¹)₂, CN, C(O)R¹, SO₂R¹, SO₃H, NO₂,
R¹ représentant un atome d'hydrogène ou un groupe alkyle en C₁-C₁₂ ou aryle en C₆-C₁₂,
en un composé de formule II 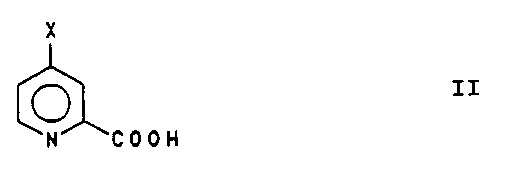 caractérisé en ce que le composé de formule I est oxydé électrochimiquement sur des anodes à NiO(OH).

2. Procédé selon la revendication 1, caractérisé en ce que l'on oxyde des composés de formule générale I dans lesquels
X représente un atome d'hydrogène ou un groupe alkyle en C₁-C₁₂, aryle en C₆-C₁₂, COOR¹, CONH₂, COHNR¹, CON(R¹)₂ ou C(O)R¹.

3. Procédé selon la revendication 1 ou 2, caractérisé en ce que l'on oxyde des composés de formule générale I dans lesquels
X représente un atome d'hydrogène ou un groupe alkyle en C₁-C₆, phényle ou naphtyle, COOR¹, CONH₂, CONHR¹, CON(R¹)₂ ou C(O)R¹.

4. Procédé selon les revendications 1 à 3, caractérisé en ce que l'oxydation s'effectue sur une anode qui consiste en du charbon ou un métal, revêtus chacun avec une couche de NiO(OH).

5. Procédé selon les revendications 1 à 4, caractérisé en ce que, pour la formation de la couche de NiO(OH) avant l'électrolyse, du nickel provenant d'une solution aqueuse de sels solubles de nickel est déposé par dépôt cathodique sur le matériau de l'électrode.

6. Procédé selon la revendication 5, caractérisé en ce que la solution de sel de nickel est ensuite remplacée par une solution aqueuse alcaline et le matériau de l'électrode est soumis à une polarisation anodique.

7. Procédé selon La revendication 6, caractérisé en ce que la cathode est constituée de nickel, palladium, platine ou acier.

8. Procédé selon les revendications 1 à 7, caractérisé en ce que l'oxydation est effectuée dans une cellule électrolytique divisée ou non divisée.

9. Procédé selon les revendications 1 à 8, caractérisé en ce que le dépôt cathodique de nickel ainsi que la polymérisation anodique sont effectués pendant 5 à 120 minutes, de préférence 20 à 40 minutes, avec une densité de courant comprise entre 1 et 100 mA/cm², de préférence entre 2 et 10 mA/cm².

10. Procédé selon les revendications 1 à 9, caractérisé en ce que l'on utilise comme électrolyte ou anolyte des solutions aqueuses qui contiennent de 0,001 à 2 moles/l d'un hydroxyde de métal alcalin.

11. Procédé selon les revendications 1 à 10, caractérisé en ce que l'on effectue l'oxydation à une température d'électrolyse comprise entre 5 et 100°C et avec une densité de courant comprise entre 1 et 50 mA/cm².
